# EUROPEAN PATENT APPLICATION

(11) **EP 1 691 200 A1**
(43) Date of publication of application: **16.08.2006**
(21) Application number: 05003114.5
(22) Date of filing: 14.02.2005
(51) Int. Cl.: G01N 33/68

(54) **The use of BNP-type peptides and ANP-type peptides for assessing the risk of suffering from a cardiovascular complication as a consequence of volume overload**

(71) Applicant: F. HOFFMANN-LA ROCHE AG, 4070 Basel (CH); Roche Diagniostics GMBH, 68305 Mannheim (DE)
(72) Inventor: Spanuth, Eberhard, 69221 Dossenheim (DE)
(74) Representative: Huhn, Michael

(57) **Abstract**

The present invention relates to the use of cardiac hormones, particularly natriuretic peptides, for diagnosing the cardiovascular risk of a patient who is a candidate for administration of a selective Cox-2 inhibitor, comprising the steps of
a) measuring, preferably in vitro, the level of a cardiac hormone,
b) diagnosing the risk of the patient by comparing the measured level to known levels associated with different grades of risk in a patient. The most preferred cardiac hormone in the context of the present invention is NT-proBNP.

## Description

The present invention relates to the use of cardiac hormones for assessing the cardiovascular risk of a patient with respect to the administration of an anti-inflammatory drug, particularly selective Cox-2 inhibitors.

An aim of modern medicine is to provide personalized or individualized treatment regimens. Those are treatment regimens which take into account a patient's individual needs or risks. Of particular importance is the cardiovascular risk or complication, particularly an unrecognized cardiovascular risk or complication.

Selective Cox-2 inhibitors are widely used potent anti-inflammatory drugs. Compared to many other anti-inflammatory drugs, they appear to cause less gastrointestinal side-effects, such as gastrointestinal bleeds or ulcers. Therefore, for many patients treatment with selective Cox-2 inhibitors is the treatment of choice.

However, it has been noted recently, that selective Cox-2 inhibitors can lead to cardiovascular complications, possibly followed by cardiac decompensation and even death. Rofecoxib (VIOXX™), a selective Cox-2 inhibitor, was only recently removed from the market voluntarily by the manufacturing company, Merck, after a 3.9 times rise in the rate of serious thromboembolic incidents had led to premature discontinuation of the APPROVE study (Adenomatous Polyp Prevention On Vioxx). Only patients without any recognisable cardiovascular risk were included in that study for the secondary prevention of colon adenomas. Even in the early post-observation phase higher blood pressure levels had been noticed with 25 mg rofecoxib than with a placebo. 16 additional cardiovascular incidents per 1,000 treated patients (myocardial infarction, stroke) with 25 mg rofecoxib compared with a placebo (Topol EJ. Failing the public health--rofecoxib, Merck, and the FDA. N Engl J Med 2004; 351: 1707-9) were noticed after 18 months of follow-up. An increased cardiovascular risk has also been suspected in other studies (VIGOR) and has been supported by data from a recent meta-analysis (Bombardier C, Laine L, Reicin A, Shapiro D, Burgos-Vargas R, Davis B, Day R, Ferraz MB, Hawkey CJ, Hochberg MC, Kvien TK, Schnitzer TJ; VIGOR Study Group. Comparison of upper gastrointestinal toxicity of rofecoxib and naproxen in patients with rheumatoid arthritis. VIGOR Study Group. N Engl J Med 2000; 343: 1520-8; Jüni P, Nartey L, Reichenbach S, Sterchi R, Dieppe PA, Egger M. Risk of cardiovascular events and rofecoxib: cumulative meta-analysis. Lancet 2004; online ahead).

Similar cardiovascular side-effects are suspected within the scope of a class effect for other selective cyclooxygenase (COX)-2 inhibitors such as celecoxib (Celebrex™, Pfizer), lumiracoxib (Prexige™, Novartis) and parecoxib, a pro-drug of valdecoxib, although at a lower incidence rate (Fitzgerald GA. Coxibs and cardiovascular disease. N Engl J Med 2004; 351: 1709-1711. Mukherjee D, Nissen SE, Topol EJ. Risk of cardiovascular events associated with selective COX-2 Inhibitors. JAMA 2001; 286: 954-959.).

Cardiovascular complications, such as acute myocardial infarction or stroke, may lead to serious health problems and even death. Therefore, many patients who would strongly benefit from treatment with selective Cox-2 inhibitors are not treated as it is unknown whether treatment may result in a cardiovascular complication.

Cardiovascular problems or risks can remain asymptomatic for long periods of time. Therefore, reliable diagnosis of the presence of a cardiovascular risk is more difficult and error-prone than generally believed. In particular, general practitioners and non-cardiologists often are not able to identify a previously unrecognized cardiovascular problem.

According to the state of the art, it is only possible to exclude patients with cardiovascular symptoms or a known history of heart disease or hypertension from treatment with selective Cox-2 inhibitors.

This risk management is insufficient, as also asymptomatic patients may develop a cardiovascular complication due to an unrecognized predisposition, e.g. the presence of an arterial plaque. As mentioned earlier, only patients without any recognisable cardiovascular risk were included in the APPROVE study, yet treatment led to cardiovascular complications in several patients.

In the prior art, no hint is given how the risk of a cardiovascular complication associated with treatment with a selective Cox-2 inhibitor can be diagnosed. Particularly, no reference has been made how such diagnosis can be made in patients that have no known history of cardiovascular complications.

This does not only apply with respect to selective Cox-2 inhibitors, but also with respect to other classes of anti-inflammatory drugs, e.g. being inhibitors of Cox-2 and other target (e.g. Cox-1), and which may cause cardiovascular complications. Examples for these other classes of anti-inflammatory drugs include non-selective Cox-2 inhibitors (compounds inhibiting Cox-1 and Cox-2). Even though the risk of leading to cardiovascular complications is not as high as for selective Cox-2 inhibitors, there may be cases where a possible cardiovascular complication has to be taken into account.

Therefore, there is a need to for a method or means to identify risk patients before they receive treatment with anti-inflammatory drugs, which drugs are preferably selective Cox-2 inhibitors. Particularly, there is a need to provide a suitable diagnostic means for the purpose of carrying out the method. Particularly, there is a need for a diagnostic means that allows to identify risk patients that have no history or no known history of a cardiovascular risk or complication. In particular, the diagnostic means should be reliable and suited for use by general practitioners and non-cardiologists.

The object of the invention is attained by a method for diagnosing the cardiovascular risk of a patient who is a candidate for administration compound having Cox-2 inhibiting properties, comprising the steps of
a) measuring, preferably in vitro, the level of a cardiac hormone,
b) diagnosing the risk of the patient by comparing the measured level to known levels associated with different grades of risk in a patient.

The compound having Cox-2 inhibiting properties can be any compound having anti-inflammatory properties. Most preferably, the compound is a selective Cox-2 inhibitor.

The method may also comprise the step of taking a body fluid or tissue sample from the patient. Within the present invention, the taking of the said body fluid or tissue sample can preferably be carried out by non-medical staff (i.e. not having an education necessary for carrying out the profession of a physician). This applies in particular when the body sample is blood.

The object of the invention is also attained by a method of deciding on the possible treatment of a patient with a compound having Cox-2 inhibiting properties, which method comprises
a) measuring, preferably in vitro, the level of a cardiac hormone in the patient,
b) comparing the measured level with known levels associated with different grades of risk in a patient,
c) optionally initiating an examination of the patient by a cardiologist,
d) recommending the initiation of the treatment or refraining from the treatment, optionally in consideration of the result of the patient's examination by the cardiologist.

The compound having Cox-2 inhibiting properties can be any compound having anti-inflammatory properties. Most preferably, the compound is a selective Cox-2 inhibitor.

The object of the invention is also attained by use of a diagnostic means for measuring, preferably in vitro, a patient's level of a cardiac hormone, particularly a natriuretic peptide, for diagnosing the cardiovascular risk of of a patient who is a candidate for administration, particularly future administration, of a compound having Cox-2 inhibiting properties. Preferably the level is determined in a body fluid or tissue sample for the patient.

The compound having Cox-2 inhibiting properties can be any compound having anti-inflammatory properties. Most preferably, the compound is a selective Cox-2 inhibitor.

The present invention provides simple and inexpensive methods and means to screen patients who are about to receive medication with a compound having Cox-2 inhibiting properties, in particular with a selective Cox-2 inhibitor, for their risk of developing a cardiovascular complication as a consequence of said medication. The present invention also provides levels of cardiac hormones indicating the existence or severity of a cardiovascular risk in patients with or without obvious symptoms.

It may be assumed that patients with a cardiovascular disorder in their case history (myocardial infarction, unstable angina pectoris, coronary artery disease, CABG, stroke) or patients with an underlying inflammatory disorder such as chronic polyarthritis, osteoarthritis, or rheumatoid arthritis are predisposed to the occurrence of cardiovascular complications. In rheumatoid arthritis, the cardiovascular death rate is particularly high for various reasons.

These use of natriuretic peptides as molecular or biochemical markers is known as such. In WO 02/089657, it has been suggested to measure brain natriuretic peptide (BNP) to diagnose various myocardial dysfunctions. In WO 02/083913 it has been suggested to use BNP to predict near-term morbidity or mortality in patients with non-ST-elevated acute coronary syndromes.

The present invention is particularly advantageous to general practitioners, specialized physicians, and specialized wards, departments, or clinics which frequently have no access to extensive cardiological examination by cardiologists. The present invention provides means and methods to such non-cardiologists for simple and reliable screening of patients and identification of those patients who are posed at a cardiovascular risk with respect to administration of a Cox-2 inhibitor, in particular a selective Cox-2 inhibitor. Preferably, the decision about continuing or initiating treatment with the Cox-2 inhibitor, in particular the selective Cox-2 inhibitor will be taken by the cardiologist. This in particular applies in cases where high levels of the natiuretic peptides are measured. In cases where the patient is diagnosed to have no cardiovascular risk, the decision to continue or initiate treatment with a selective Cox-2 inhibitor may be taken by a physician other than a cardiologist.

The invention takes advantage of certain biochemical or molecular markers. The terms "biochemical marker" and "molecular marker" are known to the person skilled in the art. In particular, biochemical or molecular markers are gene expression products which are differentially expressed (i.e. upregulated or downregulated) in presence or absence of a certain condition, disease, or complication. Usually, a molecular marker is defined as a nucleic acid (such as an mRNA), whereas a biochemical marker is a protein or peptide. The level of a suitable biochemical or molecular marker can indicate the presence or absence of the condition, disease, risk, or complication, and thus allow diagnosis.

The present invention particularly takes advantage of natriuretic peptides as biochemical markers. It should be noted that natriuretic peptides may be secreted in response to ischemia. Furthermore, taking advantage of combinations of any natriuretic peptides as biochemical markers is considered in the context of the present invention.

Natriuretic peptides according to the present invention comprise ANP-type and BNP-type peptides and variants thereof (see e.g. Bonow, R.O. (1996). New insights into the cardiac natriuretic peptides. Circulation 93: 1946-1950).

ANP-type peptides comprise pre-proANP, proANP, NT-proANP, and ANP.

BNP-type peptides comprise pre-proBNP, proBNP, NT-proBNP, and BNP.

The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP).

Preferred natriuretic peptides according to the present invention are NT-proANP, ANP, NT-proBNP, BNP, and variants thereof. ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renally. The in-vivo half-life of NTproBNP is 120 min longer than that of BNP, which is 20 min (Smith MW, Espiner EA, Yandle TG, Charles CJ, Richards AM. Delayed metabolism of human brain natriuretic peptide reflects resistance to neutral endopeptidase. J Endocrinol. 2000; 167: 239-46.).

Preanalytics are more robust with-NT-proBNP allowing easy transportation of the sample to a central laboratory (Mueller T, Gegenhuber A, Dieplinger B, Poelz W, Haltmayer M. Long-term stability of endogenous B-type natriuretic peptide (BNP) and amino terminal proBNP (NT-proBNP) in frozen plasma samples. Clin Chem Lab Med 2004; 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller T, Gegenhuber A, et al., Clin Chem Lab Med 2004; 42: 942-4, supra; Wu AH, Packer M, Smith A, Bijou R, Fink D, Mair J, Wallentin L, Johnston N, Feldcamp CS, Haverstick DM, Ahnadi CE, Grant A, Despres N, Bluestein B, Ghani F. Analytical and clinical evaluation of the Bayer ADVIA Centaur automated B-type natriuretic peptide assay in patients with heart failure: a multisite study. Clin Chem 2004; 50: 867-73.).

Therefore, depending on the time-course or properties of interest, either measurement of the active or the inactive forms of the natriuretic peptide can be advantageous. The most preferred natriuretic peptides according to the present invention are NT-proBNP or variants thereof.

The term "variants" in this context relates to peptides substantially similar to said peptides. The term "substantially similar" is well understood by the person skilled in the art. In particular, a variant may be an isoform or allele which shows amino acid exchanges compared to the amino acid sequence of the most prevalent peptide isoform in the human population. Preferably, such a substantially similar peptide has a sequence similarity to the most prevalent isoform of the peptide of at least 80%, preferably at least 85%, more preferably at least 90%, most preferably at least 95%. Substantially similar are also proteolytic degradation products which are still recognized by the diagnostic means or by ligands directed against the respective full-length peptide.

The term "variant" also relates to a post-translationally modified peptide such as glycosylated peptide. A "variant" is also a peptide which has been modified after collection of the sample, for example by covalent or non-covalent attachment of a label, particularly a radioactive or fluorescent label, to the peptide.

Other embodiments of the invention include the measuring of different cardiac hormones in combination, simultaneously or non-simultaneously. For example, measuring different cardiac hormones can yield important additional information, e.g. on the time-course of a developing cardiovascular complication. Therefore, the present invention also relates to measuring both an ANP-type peptide, or a variant thereof, and a BNP-type peptide, or a variant thereof.

Diagnosing according to the present invention includes monitoring, confirmation, subclassification and prediction of the relevant disease, complication, or risk. Monitoring relates to keeping track of an already diagnosed risk or complication, e.g. to analyze an elevation or decrease of the risk or the influence of a particular treatment on the elevation or decrease of the risk. Confirmation relates to the strengthening or substantiating a diagnosis already performed using other indicators or markers. Subclassification relates to further defining a diagnosis according to different subclasses of the diagnosed disease risk, e.g. defining according to increased risk or highly increased risk. Prediction relates to assessing a cardiovascular risk before other symptoms or markers have become evident or have become significantly altered.

Individuals suffering from a cardiovascular disease can be individuals suffering from stable angina pectoris (SAP) and individuals with acute coronary syndromes (ACS). ACS patients can show unstable angina pectoris (UAP) or these individuals have already suffered from a myocardial infarction (MI). MI can be an ST-elevated MI or a non-ST-elevated MI. The occurring of an MI can be followed by a left ventricular dysfunction (LVD). Finally, LVD patients undergo congestive heart failure (CHF) with a mortality rate of roughly 15 %.

Furthermore, individuals suffering from a cardiovascular complication can be individuals suffering from stroke. The term "stroke" relates to any cerebro-vascular event in which blood flow to small or large regions of the brain is interrupted, e.g due to haemorrhage into the brain or a thrombosis of a cerebral artery. A stroke may result in temporary loss of consciousness or paralysis. In this case, the stroke is termed "apoplectic stroke".

The individuals may show clinical symptoms (e.g. dyspnea, chest pain, see also NYHA classification below) and they may be asymptomatic. Although the present invention is particularly advantageous to identify risk patients showing no symptoms of a preexisting cardiovascular disease, risk, or complication, it is also useful in other settings, e.g to confirm or monitor the risk status of the patient showing symptoms of a cardiovascular disease.

The term "cardiovascular risk" relates to the risk of developing a cardiovascular complication.

The present invention relates to "cardiovascular complications" developing as a consequence of administration of a Cox-2 inhibitor, in particular a selective Cox-2 inhibitor. Cardiovascular complications are also known as "cardiovascular events". In the following only the term "cardiovascular complication" will be used.

The term "cardiovascular complication" is known to the person skilled in the art. Thus, a "cardiovascular complication" according to the present invention relates to any kind of such cardiovascular complication known to the person skilled in the art, particularly the term refers to a complication related to arterial thrombosis or development of arterial thrombosis. Arterial thrombosis may cause coronary syndromes (e.g. acute myocardial infarction or stroke)

Particularly, "cardiovascular complication" relates to a complication in the arterial system, e.g. ACS, UAP, MI, ST-elevated MI, non-ST-elevated MI, or stroke.

More particularly, "cardiovascular complication" relates to MI, ST-elevated MI, non-ST-elevated MI, or stroke.

Symptoms of cardiovascular diseases have been classified into a functional classification system according to the New York Heart Association (NYHA). Patients of Class I have no obvious symptoms of cardiovascular disease. Physical activity is not limited, and ordinary physical activity does not cause undue fatigue, palpitation, or dyspnea (shortness of breath). Patients of class II have slight limitation of physical activity. They are comfortable at rest, but ordinary physical activity results in fatigue, palpitation, or dyspnea. Patients of class III show a marked limitation of physical activity. They are comfortable at rest, but less than ordinary activity causes fatigue, palpitation, or dyspnea. Patients of class IV are unable to carry out any physical activity without discomfort. They show symptoms of cardiac insufficiency at rest. If any physical activity is undertaken, discomfort is increased.

A cardiovascular complication according to the present invention may eventually cause symptoms, particularly symptoms according to NYHA class II-IV, more particularly according to NYHA class III-IV.

Another characteristic of cardiovascular complication can be the "left ventricular ejection fraction" (LVEF) which is also known as "ejection fraction". People with a healthy heart usually have an unimpaired LVEF, which is generally described as above 50 %. Most people with a systolic heart disease which is symptomatic generally have an LVEF of 40 % or less.

A cardiovascular complication (e.g. MI) may eventually result in an LVEF of 40% or less.

A cardiac insufficiency may either be "compensated" or "decompensated". Compensated means that the regular oxygen need of the body can still be satisfied, whereas decompensated means that the regular oxygen need of the body is not satisfied anymore.

"Diagnosing the cardiovascular risk" according to the present invention includes diagnosing (i.e. becoming aware of) a cardiovascular risk, but also monitoring an elevation or decrease of a pre-existing or known cardiovascular risk.

The term "patient" according to the present invention relates to a healthy individual, an apparently healthy individual, or particularly an individual suffering from a disease. Particularly, the patient is suffering from or treated for rheumatism, rheumatoid arthritis (chronic polyarthritis), osteoarthritis, other inflammatory diseases, ulcerations, or cancer. In this context it should be noticed that the aforementioned diseases are diseases in which administration of compounds having Cox-2 inhibiting properties (anti-inflammatory drugs), in particular selective Cox-2 inhibitors, is considered as part of the treatment. Even more particularly, the patient has no known history of cardiovascular risk or complication, and/or no or little symptoms of a cardiovascular risk or complication, and/or he is not being treated for a cardiovascular disease, risk, or complication. However, also healthy individuals who have no signs or history of a cardiovascular risk or complication are considered to be patients according to the present invention.

Preferably, the patient is treated or is about to be treated with a selective Cox-2 inhibitor. Such a patient is understood to be a "candidate" for treatment with a selective Cox-2 inhibitor.

It is known to the person skilled in the art, under what circumstances a cardiovascular complication can be considered to occur "as a consequence" of administration of a Cox-2 inhibitor, in particular a selective Cox-2 inhibitor. It should be understood that it may not be possible or necessary to definitively establish the causal relationship. It may be sufficient to establish a sufficiently high probability that the cardiovascular complication will be precipitated by administration of the Cox-2 inhibitor, in particular the selective Cox-2 inhibitor. E.g., the APPROVE study indicates an almost four-fold increase in the number of cardiovascular complications between the test and the placebo group. For the purpose of the present invention, it may be justified to assume that any cardiovascular complication in a patient being treated with a selective Cox-2 inhibitor is also caused by the treatment.

Furthermore, the person skilled in the art is familiar with methods or signs indicating a causal relationship. For example, a time relationship between administration of the drug and the manifestation of a cardiovascular complication is always an indicator that the drug has been causal for the complication or "precipitated" the complication. E.g. the increase in the number of cardiovascular complications in the APPROVE study became evident after 18 months of treatment. In another example, if the severity of the cardiovascular complication correlates with the amount of the drug administered, it may indicate a causal relationship. For example, pathological changes in an echocardiogram or electrocardiogram may improve upon discontinuing treatment with the drug. In a particular example, the level of one or more cardiac hormones may be monitored before and during administration of the anti-inflammatory drug. If the level of the cardiac hormone increases upon beginning of treatment and decreases upon discontinuing treatment with the drug, it indicates that the drug is causing an elevation of the cardiovascular risk. Similarly, if the level correlates with the dosage of the drug, it also indicates that the drug is causing an elevation of the cardiovascular risk. Any cardiovascular complication manifesting under such circumstances is likely to be caused by the administration of the drug. This is even more likely, if the increase of the level of the cardiac hormone upon administration of the drug is unusually high compared to other patients or if a small increase in dosage causes an unusually steep increase in the level of the cardiac hormone.

Anti-inflammatory drugs are known to the person skilled in the art. Particularly, such drugs include non-steroid anti-rheumatics, Cox-2 inhibitors, corticosteroids, and TNF inhibitors.

Examples for anti-inflammatory drugs include Alclofenac; Alclometasone Dipropionate; Algestone Acetonide; Alpha Amylase; Amcinafal; Amcinafide; Amfenac Sodium; Amiprilose Hydrochloride; Anakinra; Anirolac; Anitrazafen; Apazone; Balsalazide Disodium; Bendazac; Benoxaprofen; Benzydamine Hydrochloride; Bromelains; Broperamole; Budesonide; Carprofen; Cicloprofen; Cintazone; Cliprofen; Clobetasol Propionate; Clobetasone Butyrate; Clopirac; Cloticasone Propionate; Cormethasone Acetate; Cortodoxone; Celecoxib; Rofecoxib (VIOXX); Etoricoxib; Valdecoxib; Parecoxib; lumiracoxib; Deflazacort; Desonide; Desoximetasone; Dexamethasone Dipropionate; Diclofenac; Diclofenac Potassium; Diclofenac Sodium; Diflorasone Diacetate; Diflumidone Sodium; Diflunisal; Difluprednate; Diftalone; Dimethyl Sulfoxide; Drocinonide; Endrysone; Enlimomab; Enolicam Sodium; Epirizole; Etodolac; Etofenamate; Felbinac; Fenamole; Fenbufen; Fenclofenac; Fenclorac; Fendosal; Fenpipalone; Fentiazac; Flazalone; Fluazacort; Flufenamic Acid; Flumizole; Flunisolide Acetate; Flunixin; Flunixin Meglumine; Fluocortin Butyl; Fluormetholone Acetate; Fluquazone; Flurbiprofen; Fluretofen; Fluticasone Propionate; Furaprofen; Furobufen; Halcinonide; Halobetasol Propionate; Halopredone Acetate; Ibufenac; Ibuprofen; Ibuprofen Aluminium; Ibuprofen Piconol; Ilonidap; Indomethacin; Indomethacin Sodium; Indoprofen; Indoxole; Intrazole; Isoflupredone Acetate; Isoxepac; Isoxicam; Ketoprofen; Lofemizole Hydrochloride; Lornoxicam; Loteprednol Etabonate; Meclofenamate Sodium; Meclofenamic Acid; Meclorisone Dibutyrate; Mefenamic Acid; Mesalamine; Meseclazone; Methylprednisolone Suleptanate; Morniflumate; Nabumetone; Naproxen; Naproxen Sodium; Naproxol; Nimazone; Olsalazine Sodium; Orgotein; Orpanoxin; Oxaprozin; Oxyphenbutazone; Paranyline Hydrochloride; Pentosan Polysulfate Sodium; Phenbutazone Sodium Glycerate; Pirfenidone; Piroxicam; Piroxicam Cinnamate; Piroxicam Olamine; Pirprofen; Prednazate; Prifelone; Prodolic Acid; Proquazone; Proxazole; Proxazole Citrate; Rimexolone; Romazarit; Salcolex; Salnacedin; Salsalate; Salycilates; Sanguinarium Chloride; Seclazone; Sermetacin; Sudoxicam; Sulindac; Suprofen; Talmetacin; Talniflumate; Talosalate; Tebufelone; Tenidap; Tenidap Soidum; Tenoxicam; Tesicam; Tesimide; Tetrydamine; Tiopinac; Tixocortol Pivalate; Tolmetin; Tolmetin Sodium; Triclonide; Triflumidate; Zidometacin; Zomepirac Sodium; Etanercept; Lenercept; Infliximab; Cortisone; Fluocortolone; Hydrocortisone; Methyl-prednisolone; Prednisolone; Prednisone; Prednylidene.

As the case may be, it is comprised in the present invention to measure NT-proBNP also before administering any of the above-mentioned compounds.

The term "non-steroidal anti-rheumatics" (also referred to as non-steroidal anti-inflammatory drugs or NSAIDs) is known to the person skilled in the art. NSAIDs inhibit cyclooxygenases (also known as prostaglandin-H-synthetases). Cyclooxygenases catalyze the reaction from arachidonic acid to prostaglandin H₂ (a cyclic endoperoxide), which is the precursor of prostaglandin I₂ (also known as prostacycline), thromboxan A₂, and other prostaglandins. Prostaglandins play a significant role in pain, fever, and inflammatory reactions. There are two isoforms of cyclooxygenases, Cox-1 and Cox-2. The Cox-2 gene is an immediate early gene and is induced under conditions of tissue damage, pain reactions, or inflammatory reactions. Thus, NSAIDs include Cox-1 inhibitors and Cox-2 inhibitors. The NSAIDs may inhibit both isoforms or they may be selective for one isoform (i.e. they inhibit only one of the two isoforms at the therapeutic dosage).

Examples for unspecific NSAIDs include Ibuprofen; Flurbiprofen; Naproxen; Flufenamic Acid; Mefenamic Acid; Piroxicam; Diclofenac; Phenbutazone Sodium Glycerate; Indometacin; Tenoxicam.

Selective Cox-2 inhibitors according to the present invention are compunds which, under therapeutic conditions, do inhibit expression or, preferably, the enzymatic function of Cox-2, whereas not significantly inhibiting expression or, preferably, the enzymatic function of Cox-1. Examples for selective Cox-2 inhibitors include coxibes (e.g. celecoxib, rofecoxib, etoricoxib, valdecoxib, parecoxib (a pro-drug of valdecoxib), lumiracoxib), meclofenatmate, sulindac sulphide, diclofenac, nimesulide, meloxicam, etodolac, NS398, L-745,337, DFP (3-(2-propyloxy)-4-(4-methylsulphonylphenyl)-5,5-dimethylfuranone). The latter three compounds are described in Warner, T.D., et al., 1999).

The enzymatic function of the two cyclooxygenases can be measured according to methods known in the art, including suitable in vivo or in vitro tests. A typical marker for the enzymatic function of Cox-1 is the formation of thromboxan A₂, whereas a typical marker for the enzymatic function of Cox-2 is the formation of prostaglandins (e.g. prostaglandin E₂ from macrophages.

Examples for a suitable test systems have been published (e.g. Warner, T.D., Giuliano, F., Vojnovic, I., et al. (1999). Nonsteroid drug selectivities for cyclo-oxygenase-1 rather than cyclo-oxygenase-2 are associated with human gastrointestinal toxicity: A full in vitro analysis. Proceedings of the National Academy of Sciences USA, vol. 96., pp. 7563-7568, a relevant erratum has been published in vol. 96(17), p. 9966d). This assay will be referred to as the William Harvey Modified Assay. The assay is described in detail in Warner T.D., et al. supra, on page 7563-4, the description of which is expressly incorporated herein by reference.

Preferably, a selective Cox-2 inhibitor according to the present invention is more than 5-fold Cox-2 selective according to the William Harvey Modified Assay, more preferably more than 50-fold Cox-2 selective according to the William Harvey Modified Assay (see Warner, T.D. et al., supra, Fig. 3 on page 7567).

Alternatively, the selective Cox-2 inhibitor according to the present invention is a compound preferably being more selective for Cox-2 than diclofenac, more preferably being more selective for Cox-2 than nimesulide, even more preferably at least as selective as for Cox-2 as celecoxib under therapeutic conditions.

In another preferred embodiment, the present invention relates to means and methods for diagnosing the cardiovascular risk of a patient who is a candidate for administration of a "coxibe". Examples for coxibes include celecoxib (Celebrex™, Pfizer), rofecoxib (VIOXX™, Merck), etoricoxib, valdecoxib, parecoxib (a pro-drug of valdecoxib), lumiracoxib (Prexige™, Novartis). Oth er similar compounds, several of which are under development and examination, are also included in the scope of the present invention.

The currently discussed pathomechanism for the undesired cardiovascular side-effects of selective Cox-2 inhibitors may be as follows: The inhibition of prostaglandin I₂ (PGI₂) formation is attributed a major role in the genesis of these cardiovascular incidents. PGI₂ leads to inhibition of thrombocyte aggregation, vasodilatation and prevention of the proliferation of smooth muscle cells in vitro (Fig. 2).

On the other hand, thromboxane A₂, the most important COX-1 product of platelets leads to platelet aggregation, vasoconstriction and vascular proliferation. It is suspected that the interference with the balanced equilibrium between PGI₂ and thromboxane A₂ promotes intravascular activation of hemostasis, leads to an increase in blood pressure and enhances the acceleration of atherosclerosis (4).

Examples for corticosteroids include cortisone; fluocortolone; hydrocortisone; methyl-prednisolone; prednisolone; prednisone; prednylidene.

TNF inhibitors are also used as anti-inflammatory drugs, in particular in patients with rheumatoid arthritis (increased concentrations of TNF-α were found in patients suffering from rheumatoid arthritis). Inhibition of TNF-α also reduces the formation of IL-1 and IL-6. Examples for TNF inhibitors include Etanercept, Lenercept (an analogon of Etanercept), Infliximab, and D2E7 (a completely humanized monoclonal antibody against TNF-α).

In another preferred embodiment, the present invention relates to additionally measuring the level of at least one marker chosen from the group consisting of (a) markers of inflammation, (b) markers of endothel function, (c) markers of ischemia, (d) markers of thrombocyte activation, (e) markers of atherosclerosis activation, and (f) markers of intravascular activation of coagulation.

Measuring an additional marker may increase the selectivity and specificity of diagnosis. It may also serve to confirm a diagnosis established by measuring the level of a natriuretic peptide.

Markers of inflammation according to the present invention include any markers indicative of an inflammatory process, particularly a vascular or arterial inflammatory process. Particularly, markers of inflammation according to the present invention comprise inflammatory active cytokines. Examples for markers of inflammation include interferons (e.g. interferon gamma), interleukins (e.g. IL-1, IL-6, IL-8), Tumor necrosis factor (TNF) alpha), CRP (C-reactive protein), hsCRP (high-sensitivity C-reactive protein).

Markers of endothel function according to the present invention comprise any markers indicative of intravascular repair processes, including cytokines related to such processes. Examples for such markers include selectins (e.g. E-selectin, P-selectin), ICAM-1 (intercellular cell adhesion molecule-1), VCAM-1 (vascular cell adhesion molecule-1), PDGF (platelet-derived growth factor), TGF-alpha, TGF-beta, catecholamines, prostaglandins, angiotensin, endothelin, NO (nitric oxide). Although already mentioned as being markers of inflammation, TNF-alpha, TNF-beta, and IL-1 may also be considered to belong to the group of markers of endothel function.

Markers of ischemia according to the present invention comprise any markers indicative of insufficient oxygen supply resulting in ischemia. Particularly, the insufficient oxygen supply is local and any resulting ischemia is also local. Such local ischemia may be caused by arterial thrombosis limiting the blood flow to the target tissue of the affected artery. Examples for markers of ischemia include ischemic modified albumin (IMA) and high-sensitive troponins (e.g. TnI and TnT).

Markers of thrombocyte activation according to the present invention comprise any markers indicative of activation or aggregability of blood platelets. A preferred marker is soluble CD40 ligand (SCD40L). It should be understood that thrombocyte activation or platelet aggregability may also be measured by analysis of a thrombocyte-rich blood plasma sample. After taking the sample, a trigger substance is added and the time-course of aggregation of thrombocytes is measured by the reduction in turbidity. The reduction in turbidity is caused by the aggregation of the many small platelets into larger aggregates, which reduces the light scatter. The trigger substance may be any substance deemed appropriate by the person skilled in the art (e.g. adenosine diphosphate (ADP), serotonin, collagen, protease, or ristocetine). Diagnosis is performed by comparing the result of the measurement to result of the same experiment in a control sample or control group. Therefore, such test may be considered a marker according to the present invention.

Markers of atherosclerosis activation according to the present invention comprise any markers indicative of progression of atherosclerosis. Examples for markers of atherosclerosis activation include lipoprotein-associated phospholipase A2 (Lp-PLA2).

Examples for markers of intravascular activation of coagulation include fibrinogen degradation products, D-dimer, plasminogen activator inhibitor.

Diagnosis according to the present invention is preferably done by use of a diagnostic means. A diagnostic means is any means that allows to measure the level amount, or concentration of a substance of interest, particularly a peptide or polypeptide of interest, more particularly a cardiac hormone.

Methods and diagnostic means which can be used to determine the levels of the respective peptides are known to the person skilled in the art. These methods include microplate ELISA-based methods, fully-automated or robotic immunoassays (available for example on Elecsys™ analyzers), CBA (an enzymatic Cobalt Binding Assay, available for example on Roche-Hitachi™ analyzers), and latex agglutination assays (available for example on Roche-Hitachi™ analyzers).

Furthermore, the person skilled in the art is familiar with different methods of measuring the level of a peptide or polypeptide. The term "level" relates to amount or concentration of a peptide or polypeptide in a patient (more specifically, in the blood or urine of the patient), or a sample taken from a patient (e.g. a blood or urine sample). The term "measuring" according to the present invention relates to determining the amount or concentration, preferably semi-quantitatively or quantitatively, of the nucleic acid, peptide, polypeptide, or other substance of interest. Measuring can be done directly or indirectly. Indirect measuring includes measuring of cellular responses, bound ligands, labels, or enzymatic reaction products.

In the context of the present invention, amount also relates to concentration. It is evident, that from the total amount of a substance of interest in a sample of known size, the concentration of the substance can be calculated, and vice versa.

Measuring can be done according to any method known in the art. Preferred methods are described in the following.

In one embodiment; the method for measuring the level of a peptide or polypeptide of interest, particularly a cardiac hormone, comprises the steps of (a) contacting a cell capable of a cellular response to the peptide or polypeptide with the peptide or polypeptide for an adequate period of time, (b) measuring the cellular response.

In another embodiment, the method for measuring the level of a peptide or polypeptide of interest, particularly a cardiac hormone, comprises the steps of (a) contacting a peptide or polypeptide with a suitable substrate for an adequate period of time, (b) measuring the amount of product.

In another embodiment, the method for measuring the level of a peptide or polypeptide of interest, particularly a cardiac hormone, comprises the steps of (a) contacting a peptide or polypeptide with a specifically binding ligand, (b) (optionally) removing non-bound ligand, (c) measuring the amount of bound ligand.

Preferably, the peptide or polypeptide is contained in a sample, particularly a body fluid or tissue sample, and the amount of the peptide or polypeptide in the sample is measured.

Peptides and polypeptides (proteins) can be measured in tissue, cell, and body fluid samples, i.e. preferably in vitro. Preferably, the peptide or polypeptide of interest is measured in a body fluid sample.

A tissue sample according to the present invention refers to any kind of tissue obtained from the dead or alive human or animal body. Tissue samples can be obtained by any method known to the person skilled in the art, for example by biopsy or curettage.

Body fluids according to the present invention may include blood, blood serum, blood plasma, lymphe, cerebral liquor, saliva, and urine. Particularly, body fluids include blood, blood serum, blood plasma, and urine. Samples of body fluids can be obtained by any method known in the art.

Methods to obtain cell samples include directly preparing single cells or small cell groups, dissociating tissue (e.g. using trypsin), and separating cells from body fluids, e.g. by filtration or centrifugation. Cells according to the present invention comprise also platelets and other non-nuclear cells, e.g. erythrocytes.

If necessary, the samples may be further processed. Particularly, nucleic acids, peptides or polypeptides may be purified from the sample according to methods known in the art, including filtration, centrifugation, or extraction methods such as chloroform/phenol extraction.

For measuring cellular responses, the sample or processed sample is added to a cell culture and an internal or external cellular response is measured. The cellular response may include the expression of a reporter gene or the secretion of a substance, e.g. a peptide, polypeptide, or a small molecule.

Other preferred methods for measurement may include measuring the amount of a ligand binding specifically to the peptide or polypeptide of interest. Binding according to the present invention includes both covalent and non-covalent binding.

A ligand according to the present invention can be any peptide, polypeptide, nucleic acid, or other substance binding to the peptide or polypeptide of interest. It is well known that peptides or polypeptides, if obtained or purified from the human or animal body, can be modified, e.g. by glycosylation. A suitable ligand according to the present invention may bind the peptide or polypeptide also via such sites.

Preferably, the ligand should bind specifically to the peptide or polypeptide to be measured. "Specific binding" according to the present invention means that the ligand should not bind substantially to ("cross-react" with) another peptide, polypeptide or substance present in the sample investigated. Preferably, the specifically bound protein or isoform should be bound with at least 3 times higher, more preferably at least 10 times higher and even more preferably at least 50 times higher affinity than any other relevant peptide or polypeptide.

Non-specific binding may be tolerable, particularly if the investigated peptide or polypeptide can still be distinguished and measured unequivocally, e.g. according to its size on a Western Blot, or by its relatively higher abundance in the sample.

Binding of the ligand can be measured by any method known in the art. Preferably, the method is semi-quantitative or quantitative. Suitable methods are described in the following.

First, binding of a ligand may be measured directly, e.g. by NMR or surface plasmon resonance.

Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot).

For measurement of enzymatic reaction products, preferably the amount of substrate is saturating. The substrate may also be labeled with an detectable lable prior to the reaction. Preferably, the sample is contacted with the substrate for an adequate period of time. An adequate period of time refers to the time necessary for an detectable, preferably measurable amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand.

Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.)

The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus.

Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels.

Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously.

Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molcular Probes (Oregon). Also the use of quantum dots as fluorescent labels is contemplated.

Typical radioactive labels include ³⁵S, ¹²⁵I, ³²P, ³³P and the like. A radioactive label can be detected by any method known and appropriate, e.g. a light-sensitive film or a phosphor imager.

Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

Preferred ligands include antibodies, nucleic acids, peptides or polypeptides, and aptamers, e.g. nucleic acid or peptide aptamers. Methods to such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display.

The term "antibody" as used herein includes both polyclonal and monoclonal antibodies, as well as fragments thereof, such as Fv, Fab and F(ab)₂ fragments that are capable of binding antigen or hapten. The present invention also includes "humanized" hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art.

In another preferred embodiment, the ligand, preferably chosen from the group consisting of nucleic acids, peptides, polypeptides, more preferably from the group consisting of nucleic acids, antibodies, or aptamers, is present on an array.

Said array contains at least one additional ligand, which may be directed against a peptide, polypeptide or a nucleic acid of interest. Said additional ligand may also be directed against a peptide, polypeptide or a nucleic acid of no particular interest in the context of the present invention. Preferably, ligands for at least three, preferably at least five, more preferably at least eight peptides or polypeptides of interest in the context of the present invention are contained on the array.

According to the present invention, the term "array" refers to a solid-phase or gel-like carrier upon which at least two compounds are attached or bound in one-, two- or three-dimensional arrangement. Such arrays (including "gene chips", "protein chips", antibody arrays and the like) are generally known to the person skilled in the art and typically generated on glass microscope slides, specially coated glass slides such as polycation-, nitrocellulose- or biotin-coated slides, cover slips, and membranes such as, for example, membranes based on nitrocellulose or nylon.

The array may include a bound ligand or at least two cells expressing each at least one ligand.

It is also contemplated to use "suspension arrays" as arrays according to the present invention (Nolan JP, Sklar LA. (2002). Suspension array technology: evolution of the flat-array paradigm. Trends Biotechnol. 20(1):9-12). In such suspension arrays, the carrier, e.g. a microbead or microsphere, is present in suspension. The array consists of different microbeads or microspheres, possibly labeled, carrying different ligands.

The invention further relates to a method of producing arrays as defined above, wherein at least one ligand is bound to the carrier material in addition to other ligands.

Methods of producing such arrays, for example based on solid-phase chemistry and photo-labile protective groups, are generally known (US 5,744,305). Such arrays can also be brought into contact with substances or substance libraries and tested for interaction, for example for binding or change of confirmation. Therefore, arrays comprising a peptide or polypeptide as defined above may be used for identifying ligands binding specifically to said peptides or polypeptides.

Furthermore, it is contemplated to use so called point-of-care or lab-on-a-chip devices for obtaining the sample and measurement of the marker. Such devices may be designed analogously to the devices used in blood glucose measurement. Thus, a patient will be able to obtain the sample and measure the marker without immediate assistance of a trained physician or nurse.

Means suitable for measuring the expression level of a marker according to the present invention, such as antibodies, aptamers, antisense nucleic acids etc. have been already been described in detail earlier in this specification. In a preferred embodiment, the means is packed as a kit comprising a container for the means or agent for measurement as well as containers for any auxiliary agents for measurement, e.g. suitable buffers, filters, columns, enyzmes, etc..

In another preferred embodiment, it is contemplated that the means is a lab-on-a-chip device or any other device suitable for point-of-care diagnosis. Devices for point-of-care diagnosis are known in the art. Preferably, such a device comprises a sampling unit (e.g. for taking a blood sample) and a measurement unit (e.g. for measuring the binding of the marker to a ligand). The means may also be a test strip or other auxiliary tool for measuring the expression level of the marker in such a device.

Therefore, in another embodiment, the present invention also relates to a kit, a lab-on-chip device, or a point-of-care diagnostic device for measuring the expression level of XCR1.

The method according to the present invention comprises the step of diagnosing the risk of the patient by comparing the measured level to known levels associated with different grades of risk in a patient.

The person skilled in the art is able to determine known levels of cardiac hormones which are associated with different grades of cardiovascular risk of with respect to administration of an anti-inflammatory drug, particularly a selective Cox-2 inhibitor. In general, the higher the level of the cardiac hormone, the higher is the risk for the patient.

According to the present invention, the term "risk" relates to the probability of a particular incident, more particularly a cardiovascular complication, to take place. The grade of risk can be increased, or highly increased. The grade of risk can also not be increased. "No increased risk" means that there is apparently no risk of a cardiovascular risk with respect to administration of an anti-inflammatory drug, particularly a selective Cox-2 inhibitor.

Guidance as to what levels are associated with which grade of risk can be drawn from levels of cardiac hormones known to be associated with the presence or severity of a cardiovascular disease. For example, based on a 97.5 percentile obtained in individuals below the age of 50, a plasma level of 125 pg/ml of NT-proBNP may be considered a normal level. Higher levels of NT-proBNP correlate for example with the level of symptoms according to the NYHA classification and with the level of impairment of LVEF. The term "plasma level" relates to levels of NT-proBNP measured in blood plasma.

Below, plasma levels of NT-proBNP are given which are typically considered to be associated with the indicated grades of cardiovascular risk with respect to administration of a Cox-2 inhibiting compound as an anti-inflammatory drug, particularly a selective Cox-2 inhibitor.

It is evident, that the levels given below can serve only as a first classification of the risk of a patient. The person skilled in the art is able to determine other relevant levels from the literature (e.g. Wang, T.J., Larson, M.G., Levy, D., et al. (2004) Plasma natriuretic peptide levels and the risk of cardiovascular events and death. N Engl J Med, vol. 350, pp. 655-63; Olson, M.H., Wachtell, K. Tuxen, C., Fossum, E. et al. (2004) N-terminal pro-brain natriuretic peptide predicts cardiovascular events in patients with hypertension and left hypertrophy: a LIFE study. J Hypertens, vol. 22, pp. 1597-1604) or from other clinical studies.

Typically, a plasma level of less than 125 pg/ml of NT-proBNP is associated with no increased cardiovascular risk with respect to administration of Cox-2 inhibiting compound, particularly a selective Cox-2 inhibitor.

Typically, a plasma level from 125 to 500 pg/ml of NT-proBNP is associated with an increased cardiovascular risk with respect to administration of Cox-2 inhibiting compound, particularly a selective Cox-2 inhibitor. However, also a level between 80 and 125 pg/ml should warrant further clarification of whether an increased risk is present.

Typically, a plasma level more than 500 pg/ml of NT-proBNP is associated with a highly increased cardiovascular risk with respect to administration of a Cox-2 inhibiting compound, particularly a selective Cox-2 inhibitor. However, also a level of more than 400 pg/ml should warrant further clarification of whether a highly increased risk is present.

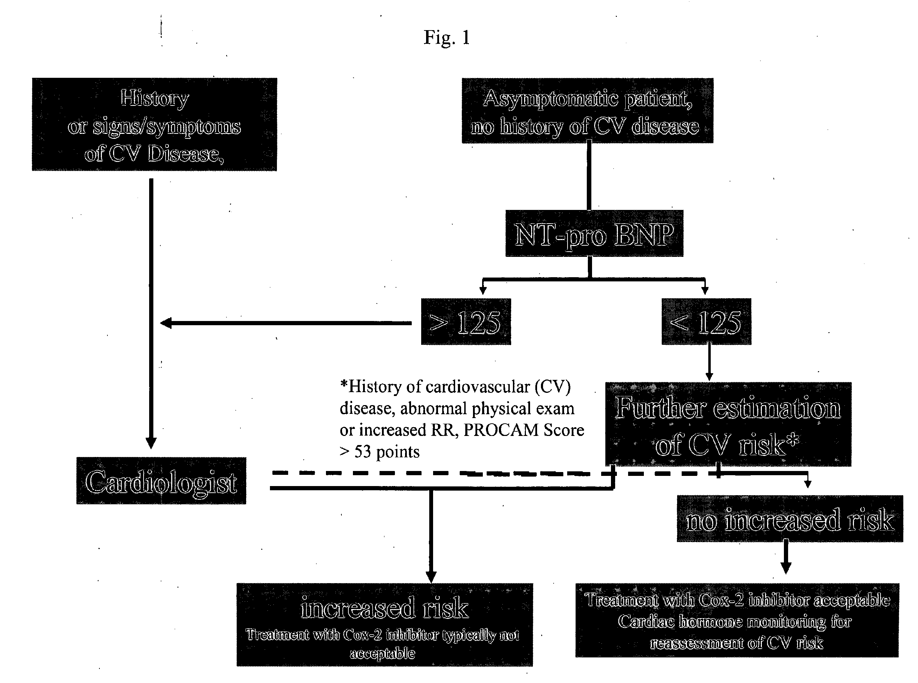

Once the risk in a patient has been diagnosed, it may have consequences for the subsequent treatment as described below (a non-limiting example for such treatment decisions is also given in Fig. 1). The grades of risk mentioned below particularly refer to the grades of risk associated with the above described levels of NT-proBNP.

If a method according to the present invention indicates no increased risk, then the anti-inflammatory drug may be administered, preferably taking into account any other known risk factors for cardiovascular disease. Preferably, administration of the drug is accompanied by further monitoring of the level of the cardiac hormone, particularly in case of high dosage or long-term application of the drug. Thus, it will be possible to early detect any unusual increase in the level of the cardiac hormone which would indicate an elevation of the cardiovascular risk.

If a method according to the present invention indicates an increased risk, then the patient has to be investigated intensively by further diagnosis according to methods known to the skilled cardiologist, such as electrocardiography, echocardiography. Treatment with a selective Cox-2 inhibitor should only be initiated upon careful consideration of risk and potential benefit.

Ttreatment may also be accompanied by further measures such as limiting or reducing the dosage of an anti-inflammatory drug administered or about to be administered, restriction of salt intake, regular moderate exercise, providing influenzal and pneumococcal immunization, surgical treatment (e.g. revascularization, ballon dilatation, stenting, by-pass surgery), administering drugs such as diuretics (including co-administration of more than one diuretic), ACE (angiotensin converting enzyme) inhibitors, β-adrenergic blockers, aldosteron antagonists, calcium antagonists (e.g. calcium channel blockers), angiotensin-receptor blockers, digitalis and any other measures known and deemed appropriate by the person skilled in the art.

The present invention also comprises a method of monitoring the treatment with a Cox-2 inhibiting compound, in particular a selective Cox-2 inhibitor, wherein the level of a cardiac hormone, in particular NT-proBNP, is measured.

If, based upon consideration of risk and potential benefit, treatment with a Cox-2 inhibiting compound, in particular a selective Cox-2 inhibitor is initiated, it may also be as an intermittent therapy. Measuring a natriuretic peptide may then be used to monitor therapy, particularly intermittent therapy, and/or to identify an elevation of the risk. The administration is stopped when the level of cardiac hormone reaches a certain value and is optionally re-initiated when the level falls below a certain value. The respective values of the cardiac hormone are those mentioned beforehand.

From the above it is evident that the present invention also provides a method of monitoring of a patient who is being treated or who is about to be treated with a Cox-2 inhibiting compound, in particular a selective Cox-2 inhibitor.

If a method according to the present invention indicates a highly increased risk, then treatment may be adapted as described for increased risk. However, administration of a Cox-2 inhibiting compound, in particular a selective Cox-2 inhibitor will *a priori* not be a treatment option for a patient with a highly increased risk. If, for whatever the reason may be, a Cox-2 inhibiting compound, in particular a selective Cox-2 inhibitor is administered, it should only be done under careful medical supervision, in particular including measuring of a natriuretic peptide at short intervals to monitor the risk and/or to identify an elevation of the risk.

### Figure Legends

- Fig. 1: Flow chart of an example of the invention. Patients who are candidates for receiving an anti-inflammatory drug (e.g. a Cox-2 inhibitor) are grouped into patients without and patients with symptoms of cardiovascular disease (CV). If the measured plasma level of NT-proBNP is above the chosen cut-off of 125 pg/ml, the patient is referred to a cardiologist. If the measured level of the cardiac hormone is below the chosen cut-off (e.g. a plasma level of 125 pg/ml NT-proBNP), the patient is subjected to a limited physical examination (e.g. establishing the PROCAM score or Framingham score, anamnesis, blood pressure (RR), possibly electrocardiogram). If the result from the physical examination does not indicate a cardiovascular risk, the anti-inflammatory drug is administered, but the patient receives monitoring of the level of the cardiac hormone. The cardiologist plays a major role in evaluating all risk indicators and treatment options.

The following examples illustrate the invention and are not intended to limit its scope in any way.

### Example 1

### Measurement of NT-proBNP:

NT-proBNP can be determined by an electrochemoluminescence immunoassay (Elecsys proBNP sandwich immuno assay; Roche Diagnostics, Mannheim, Germany) on Elecsys 2010. The assay works according to the electrochemoluminescence sandwich immunoassay principle. In a first step, the biotin-labelled IgG (1-21) capture antibody, the ruthenium-labelled F(ab')₂ (39-50) signal antibody and 20 microliters of sample are incubated at 37 °C for 9 minutes. Afterwards, streptavidin-coated magnetic microparticles are added and the mixture is incubated for additional 9 minutes. After the second incubation, the reaction mixture is transferred to the measuring cell of the system where the beats are magnetically captured onto the surface of an electrode. Unbound label is removed by washing the measuring cell with buffer.

In the last step, voltage is applied to the electrode in the presence of a tri-propylamine containing buffer and the resulting electrochemoluminescent signal is recorded by a photomultiplier. All reagents and samples are handled fully automatically by the Elecsys® instrument. Results are determined via a calibration curve which is instrument-specifically generated by 2-point calibration and a master curve provided via the reagent barcode. The test is performed according to the instructions of the manufacturer.

Blood for hormone analysis may be sampled in EDTA-tubes containing 5000 U aprotinine (Trasylol, Beyer, Germany) and Lithium-Heparin-tubes (for clinical chemistry), as appropriate. Blood and urine samples are immediately spun for 10 min. at 3400 rpm at 4 ° C. Supernatants are stored at -80 °C until analysis.

### Measurement of NT-proANP:

NT-proANP can be determined by a competitive-binding radioimmuno assay with magnetic solid phase technique in a modification of Sundsfjord, J.A., Thibault, G., et al. (1988). Idenfication and plasma concentrations of the N-terminal fragment of proatrial natriuretic factor in man. J Clin Endocrinol Metab 66:605-10., using the same rabbit-anti-rat proANP polyclonal serum, human proANP (1-30) from Peninsula Lab (Bachem Ltd, St. Helene, UK) as the standard, and iodined, proANP 1-30 purified by HPLC for radio labelling. In order to achieve high sensitivity and good precision, Dynabeads M280 with sheep-anti-rabbit IgG (Dynal Biotech, Oslo, Norway) as solid phase and second antibody may be used.

### Example 2

A 70-year-old patient suffers from severe rheumatoid arthritis and has received long-term treatment with ibuprofen. During the ibuprufen treatment he has suffered from gastrointestinal side-effects including gastrointestinal bleeds. The physician considers to change treatment to the use of selective Cox-2 inhibitors and performs a measurement of NT-proBNP. The NT-proBNP level is 223 pg/ml. The patient is referred to a cardiologist for further diagnosis. By means of echocardiogram, the cardiologist diagnoses an ischemic function defect and a risk for therapy with selective Cox-2 inhibitors. Since the cardiac function defect is not severe, an intermittent therapy with a selective Cox-2 inhibitor is initiated under close monitoring of NT-proBNP.

### Example 3

A 55-year-old female patient has been treated for 6 years for diabetes type II and is suffering from a chronic painful rheumatic disease. Therefore, her physician considers treatment with Cox-2 inhibitors. The NT-proBNP value is measured and determined to be 647 pg/ml. The patient is referred to a cardiologist for further diagnosis. Due to the high NT-proBNP value and due to the co-morbidity of diabetes, treatment with Cox-2 inhibitors is not initiated.

### Example 4

A 72-year-old patient healthy and smoker, but having no further traditional risk factors for heart disease, suffers from strong joint pain in the knees (probably due to long-time sport activities). The patient knows that his stomach is very sensitive. Therefore, treatment with selective Cox-2 inhibitors is considered. The measured plasma NT-proBNP value is determined to be 65 pg/ml. As the PROCAM score is below 40 and the only traditional risk factor is smoking, treatment with selective Cox-2 inhibitors is initiated.

## Claims

1. A method for diagnosing the cardiovascular risk of a patient who is a candidate for administration of a Cox-2 inhibiting compound, comprising the steps of
a) measuring the level of a cardiac hormone,
b) diagnosing the risk of the patient by comparing the measured level to known levels associated with different grades of risk in a patient.

2. The method according to claim 1, wherein the cardiac hormone is a natriuretic peptide.

3. The method according to claim 2, wherein the cardiac hormone is an ANP-type peptide or a variant thereof and/or a BNP-type peptide or a variant thereof.

4. The method according to claim 3, wherein the cardiac hormone is a BNP-type peptide from the group BNP and NT-proBNP and variants thereof.

5. The method according to any of claims 1 to 4, wherein the Cox-2 inhibiting compound is a selective Cox-2 inhibitor.

6. The method according to any of claims 1 to 5, wherein the cardiac hormone is NT-proBNP and a plasma level of less than 80 pg/ml of NT-proBNP is associated with no increased risk of suffering from a cardiovascular complication.

7. The method according to claim 6, wherein the plasma level is less than 125 pg/ml.

8. The method according to any of claims 1 to 5, wherein a plasma level of more than 125 and less than 500 pg/ml of NT-proBNP is associated with an increased risk of suffering from a cardiovascular complication.

9. The method according to any of claims 1 to 5, wherein a plasma level is more than 500 pg/ml of NT-proBNP is associated with an increased risk of suffering from a cardiovascular complication.

10. The method according to any of claims 1 to 9, wherein the method is carried out within the monitoring of a therapy with a selective Cox-2 inhibitor.

11. The method according to claim 10, wherein the method is for monitoring of an intermittent therapy with a selective Cox-2 inhibitor.

12. The method according to claim 11, wherein the administration is stopped when the level of cardiac hormone reaches a certain value and is optionally re-initiated when the level falls below a certain value.

13. The method according to any of claims 1 to 12, wherein the Cox-2 inhibiting compound is chosen from the group consisting of celecoxib, rofecoxib, etoricoxib, valdecoxib, parecoxib, and lumiracoxib.

14. The method according to any of claims 1 to 13, wherein additionally the level(s) of at least one marker chosen from the group consisting of
a) markers of inflammation
b) markers of endothel function
c) markers of ischemia
d) markers of thrombocyte activation
e) markers of atherosclerosis activation
f) markers of intravascular activation of coagulation
is measured.

15. The method according to any of claims 1 to 14, wherein the cardiovascular complication the risk of which is diagnosed is coronary heart disease, stable angina pectoris, acute coronary syndrome, unstable angina pectoris, myocardial infarction, ST-elevated myocardial infarction, non ST-elevated myocardial infarction, or stroke.

16. The method according to any of claims 1 to 15, wherein the level of the cardiac hormone is measured in a urine, blood, blood plasma, or blood serum sample.

17. A method of deciding on the possible treatment of a patient with a compound having Cox-2 inhibiting properties, which method comprises
a) measuring, preferably in vitro, the level of a cardiac hormone in the patient,
b) comparing the measured level with known levels associated with different grades of risk in a patient,
c) optionally initiating an examination of the patient by a cardiologist,
d) recommending the initiation of the treatment or refraining from the treatment, optionally in consideration of the result of the patient's examination by the cardiologist.

18. The use of a diagnostic means capable of measuring a patient's level of a cardiac hormone, preferably a natriuretic peptide, for diagnosing the cardiovascular risk of a patient who is a candidate for administration of a selective Cox-2 inhibitor or for monitoring the cardiovascular risk in a patient who is being treated with a selective Cox-2 inhibitor, wherein the level of a cardiac hormone is measured in a body fluid or tissue sample of the patient.

19. The use according to claim 18, wherein the cardiac hormone is a natriuretic peptide.

20. The method according to any of claims 18 to 19, wherein the level of the cardiac hormone is measured using a specifically binding ligand, an array, a microfluidic device, a chemiluminescence analyzer, or a robotic device.

21. The method according to claim 20, wherein the specifically binding ligand is an antibody or an aptamer.
